# EUROPEAN PATENT SPECIFICATION

(11) **EP 4 274 493 B1**
(45) Date of publication and mention of the grant of the patent: **09.10.2024**
(21) Application number: 22734085.8
(22) Date of filing: 13.06.2022
(51) Int. Cl.: A61B 17/12, A61F 2/24, A61F 2/966, A61B 90/00

(54) **IMPLANT DETACHMENT MECHANISM**
IMPLANTAT-ABLÖSUNGSMECHANISMUS
MÉCANISME DE DÉTACHEMENT D'IMPLANT

(30) Priority: 21.03.2022 IN 202221015511
(43) Date of publication of application: 15.11.2023
(73) Proprietor: Sahajanand Medical Technologies Limited, Surat, Gujarat 395004 (IN)
(72) Inventor: SINGHVI, Abhijeet, Valsad, Gujarat 396105 (IN); SOLANKI, Chirag Maheshbhai, Surat, Gujarat 395009 (IN); PANDEY, Shivanshu, Kanpur, Uttar Pradesh 208021 (IN); MUNJANI, Akashkumar Ashokbhai, Surat, Gujarat 395004 (IN); VASHISHT, Isha, Gurugram, Haryana 122001 (IN)
(74) Representative: Lavoix
(86) International application number: PCT/IN2022/050540
(87) International publication number: WO 2023/181048

(56) References cited:
- US-A- 5 746 769
- US-A1- 2007 135 826
- US-A1- 2009 163 934
- US-A1- 2019 009 057

## Description

The present invention relates to an implant detaching mechanism to detach an implant from an implant holder. Specifically, the invention is related to detachment mechanism for assisting in deployment of a coronary or a peripheral implant.

### BACKGROUND OF THE INVENTION

Healthy and proper functioning of organs e.g. heart, brain, liver, pancreas, kidneys, cardiovascular system etc., along with their internal parts e.g. for a heart its arteries, veins, valves, nodes, walls, and remaining constituents, is essential for healthy living and well-being of a human or an animal. However, due to factors like age, disease, infections or genetic disorder, the working efficiency of the organs reduce significantly and many times that results in a potentially life-threatening condition. Conventionally, surgery was one main option to operate on the severely diseased organ e.g., replacing the diseased heart valve by a mechanical heart valve or bypassing or removing a blocked artery using a harvested artery etc. However, in recent years, alternative less invasive transcatheter-based approaches have been developed where a percutaneous catheter delivers an implant transvascularly through variety of access points in a cardiovascular network e.g., through femoral artery, transapically, transaortic, trans-axillary etc. These implants may be, but not limited to, a stent, a valve, a mesh, a balloon, a patch, a drug-containing matrix, a shunt, or a combination thereof.

During a transvascular procedure, a catheter delivery system, carrying an implant, plays a vital role as the operator's maneuvering actions at proximal end (handle) of the delivery system directly impacts the positioning, movement of the distal section (tip and capsule), and performance of the implant after the deployment. The effect of maneuvering actions transfers through a catheter shaft from the proximal end to the distal end. The catheter shaft is situated between the proximal end and the distal end. However, sometimes, the implant doesn't get detached from the delivery system quickly and requires additional maneuvering that consumes additional time and may also reduce accuracy in positioning of the implant.
US 2019/0009057 A1 discloses an interventional medical system including an interventional medical device, a delivery cable and a locking sleeve. The device includes a plug head portion including a solid connection section and locking slots formed in the side wall of the connection section. The delivery cable includes locking pieces which extend approximately away from the central axial line of a cable head portion of the delivery cable. Distal end sections of the locking pieces are slightly closer to the central axial line of the cable head portion than their adjacent sections, so that the distal end sections of the locking pieces enter well the locking slots to achieve the connection between the plug head portion and the delivery cable. The locking sleeve is a hollow tubular body, which surrounds the outside of the delivery cable and is movable relative to the axial direction of the delivery cable. When moving relative to the axial direction of the locking sleeve, the locking pieces of the delivery cable are radially restrained by the locking sleeve. When the interventional medical system is in a locked state, namely when the plug head portion is connected with the cable head portion, the locking pieces are radially restrained by the locking sleeve such that they are limited from an expanded state into a cylindrical state (namely a compressed state), and are clamped into the locking slots. As the length of the distal end of each locking piece along the circumferential direction of the cable head portion is greater than that of the proximal end of the locking piece along the circumferential direction of the cable head portion, connection is formed between the device and the delivery cable, wherein the device and the delivery cable may not relatively move in both the axial direction and the circumferential direction; when the locking sleeve moves relative to the delivery cable towards a direction away from the device until the locking pieces elastically move away from the locking slots, the device is separated from the delivery cable. Therefore, a releasing process of the device is as follows: in a locked state, the delivery cable is stationary, and the locking sleeve is moved in the direction away from the device; the locking pieces having elasticity are gradually withdrawn from the restriction of the locking sleeve to restore preset horn shapes such that the locking pieces elastically move away from the locking slots gradually; and when the locking pieces have completely and elastically moved away from the locking slots, the device is separated from the delivery cable, thus fulfilling the objective of releasing the device

Hence, there is a need to provide a detachment mechanism in a catheter delivery system for trans-vascularly deliver an implant to avoid the shortcomings known in the art and specifically to provide a catheter delivery system that gives precision and efficiency in detachment and deployment of the implants.

### SUMMARY OF THE INVENTION

The invention is defined in the appended claims and is illustrated, according to various aspects described below.

According to the invention, an implant detaching mechanism to detach an implant from an implant holder, comprises a catheter having an inner shaft, a catheter shaft, a capsule, and a handle. The implant holder is attached to the inner shaft and having at least one pin on its peripheral surface to engage an implant to the implant holder. The catheter shaft is movable in the longitudinal direction and a capsule is attached to the distal end of the catheter shaft wherein, on movement of the catheter shaft, the capsule covers and uncovers the implant holder and at least the engaged implant partially. A thin strip, elastic in nature, is attached to the implant holder and placed between the peripheral surface of the implant holder and the engaged implant. On moving the capsule to cover the implant holder, a compressive force is being applied on the thin strip and the engaged implant. On moving the capsule to uncover the implant holder, release of potential energy stored in the thin strip causes detachment of the engaged implant from the pin. The thin strip has an eyelet or an elongated slot on its peripheral surface to accommodate overlapping portion of the pin on compression due to the movement of the capsule. The thin strip has an eyelet or an elongated slot on its peripheral surface to accommodate overlapping portion of the pin on compression due to the movement of the capsule.

The above aspects are further illustrated in the figures and described in the corresponding description below. It should be noted that the description and figures merely illustrate principles of the present invention. Therefore, various arrangements that encompass the principles of the present invention, although not explicitly described or shown herein, may be devised from the description, and are included within its scope.

### BRIEF DESCRIPTION OF ACCOMPANYING DRAWINGS

The detailed description is described with reference to the accompanying figures.
FIG. 1 illustrates an isometric view of a catheter delivery system, according to an embodiment of the present invention;
FIG. 1A illustrates a detailed view of a capsule assembly showing position of an implant holder in a catheter delivery system, according to an embodiment of the present invention;
FIG. 2 and 2A illustrate, a detailed isometric view and a detailed side view respectively, depicting an implant detachment mechanism in an implant holder of a catheter delivery system, according to an embodiment of the present invention;
FIG. 3 illustrates a detailed isometric view of an implant detachment mechanism in an implant holder in a catheter delivery system, according to another embodiment of the present invention;
FIG. 4 illustrates a detailed isometric view of an implant detachment mechanism in an implant holder in a catheter delivery system, according to yet another embodiment of the present invention;
FIG. 5 and 5A illustrate, a detailed isometric view and a detailed side view respectively, depicting an implant detachment mechanism in an implant holder of a catheter delivery system, according to yet another embodiment of the present invention;
FIG. 6 and 6A illustrate, a detailed isometric view and a detailed side view respectively, depicting an implant detachment mechanism in an implant holder of a catheter delivery system, according to yet another embodiment of the present invention;
FIG. 7 and 7A illustrate, a detailed isometric view and a detailed side view respectively, depicting an implant detachment mechanism in an implant holder of a catheter delivery system, according to yet another embodiment of the present invention; and
FIG. 8 and 8A illustrate, a detailed isometric view and a detailed side view respectively, depicting an implant detachment mechanism in an implant holder of a catheter delivery system, according to yet another embodiment of the present invention.

### DETAILED DESCRIPTION OF THE INVENTION

According to the present disclosure, in some embodiments, a catheter delivery system, for trans-vascularly delivering and deploying an implant in a human heart, comprises a detachment mechanism to ensure disconnect or detachment of the implant from the catheter delivery system once the implant reaches its deployment location and it is in correct positioning too. By definition, the detachment of the implant from the catheter delivery system is an incident after which the implant cannot be maneuvered anymore and the next process steps of retraction of the catheter from the deployment site initiate.

In one non-limiting aspect, a typical catheter delivery system comprises a distal section, a middle section, and a proximal section. The proximal section remains outside the human body and comprises a handle housing that encompasses mechanisms to control the movements at the distal section of the catheter. The distal section comprises a tip, an inner shaft, a guidewire shaft, an implant holder, and a capsule wherein, in a loaded state, the distal section comprises an implant too. The middle section is connected proximally with the handle housing and distally it connects to the distal section.

The capsule is a hollow, cylindrical structure that is movable through movement mechanisms present in the middle section and actuated from the proximal section. The capsule provides an inside space where the implant is loaded in compressed form and the capsule helps in retaining the implant in compressed form. Usually, capsule-based catheter delivery systems are used for delivery of implants whose frame structure is made of shape memory alloys e.g., Nitinol. Such implants don't require any external force to regain their un-compressed structure. Due to shape-memory property, such implants start attaining their normal structure from an end once the capsule is moved to uncover the implant, starting from the end. The implant is situated over the guidewire shaft and in between the tip and the implant holder. The inner shaft extends longitudinally along the middle section from a proximal end of the distal section and further extends till a proximal end of the proximal section. The guidewire shaft extends from the proximal end of the proximal section till a distal end of the distal section.

The implant holder is a hub-like cylindrical structure fixed on the inner shaft and situated inside the capsule at a proximal end of the distal section. The implant holder has a plurality of pins on its peripheral surface. These pins are, optionally, at equal distance and angle from each other circumferentially. These pins are to get engaged with frame of the implant at the time of loading the implant and to get disengaged at the time of deployment. As mentioned above, in some cases and due to various reasons, the implant doesn't get disengaged quickly and requires additional maneuvering to get the implant dislodged. This increases procedure time, and the positioning of the implant may also get affected.

The present disclosure utilizes a combination of design and material properties to address this issue of ensuring disengagement of the implant. The solution involves use of an elastic polymeric or metallic material whose thin strip is being placed between the peripheral surface of the implant holder and an engaging part of the frame of the implant. Preferably, the thin strip placement is around the pin of the implant holder. The thin strip is designed in such a way so that it acts as a cantilever or similar to a leaf spring whose ends are fixed. Also, in normal state, the distance between the peripheral surface of the implant holder and at least a portion of the thin strip is almost equal or lower than or higher than the height of the pin situated on the peripheral surface of the implant holder.

On loading the implant on the guidewire shaft and inside the capsule, the engaging part of the frame of the implant gets engaged with the pin of the implant holder and by design the portion of the thin strip is placed between the peripheral surface of the implant holder and the engaging part of the frame of the implant. In the implant loading procedure, the capsule is moved to compress and house the implant inside the hollow cylindrical structure of the capsule. In the process, the thin strip is also compressed, and potential energy is stored in the thin strip.

During deployment procedure, the capsule is moved to uncover the implant and the engaged part of the implant moves back to its original shape. In normal operation, this is sufficient to disengage the implant from the frame holder. However, the thin strip also tries to move back to its original position due to its elastic nature of the material used or by releasing the stored potential energy. This additional movement of the thin strip takes place simultaneously along with the disengagement of the engaged part of the implant and provides additional force to the engaged part for disengagement. In some cases, the engaged part of the frame of the implant doesn't get disengaged on removal of the compressive force applied by the capsule. In such scenario, due to material property, the thin strip applies the additional force on the engaged part of the frame of the implant and the combined force of the additional force, due to potential energy release in the thin strip, and the shape memory property of the implant frame ensures disengagement of the implant from the catheter delivery system.

According to an embodiment of the present disclosure, the thin strip can be a designed as a cantilever where one end of the thin strip is attached to a proximal end of the implant holder and the other end is free. In another embodiment, one end of the thin strip is attached to the distal end of the implant holder and the other end is free. In both the cases, the length of the thin strip is sufficiently long to cover or exceed at least half of the width of the pin. According to yet another embodiment, both the ends of the thin strip are fixed to the proximal end and the distal end of the implant holder respectively. However, the middle part of the thin strip is not connected to any surface. Also, the overall length of the thin strip is slightly higher than the distance between the points where the thin strip joins the implant holder. This gives the thin strip a bell-shaped configuration where a portion of the thin strip or the formed bell is near the pin and the height of a portion of the thin strip is almost equal or lower than or higher than the height of the pin.

According to yet another embodiment of the present disclosure, a combination of a plurality of the thin strips can be used which will act as combined cantilevers. In this embodiment, the plurality of thin strips in cantilever configuration are disposed parallel to each other or one thin strip superimposes another thin strip partially or completely. The length and location of the plurality of the thin strips may vary over peripheral surface of the implant holder. In one such embodiment, a second thin strip is situated between the proximal end of the implant holder and the distal end of the implant holder. One end of the second thin strip is attached to the peripheral surface of the implant holder whereas another end is free and, optionally, touches the superimposing or larger thin strip anywhere between the other free end of the thin strip and the joined end. The objective of the second thin strip is to provide additional force for detachment.

According to yet another embodiment of the present disclosure, a nested combination of the plurality of thin strips can be used i.e., the thin strip comprises at least a third thin strip that is attached to the thin strip and extends in space between the thin strip and the peripheral surface of the implant holder. During the procedure, the third thin strip provides additional force to the thin strip to cause detachment of the engaging part of the frame from the implant holder.

According to yet another embodiment of the present disclosure, the thin strip is designed as a cantilever where one end of the thin strip is attached to a proximal end of the implant holder and the other end is free. The thin strip has at least one step change curvature along the length of the cantilever. Due to the step-change curvatures, while being compressed by the capsule, the overall profile of the thin strip may increase in radial direction resulting in overall increase of profile of the capsule. A slot is created in the inner shaft to partially accommodate the thin strip, specifically, the middle portion of the thin strip, resulting in reduction of the overall profile of the thin strip in radial direction. The slot may be longitudinally extending or circumferentially extending. Also, the shape of the slot may be selected from, but not limited to, the rectangular, circular, helical, ovel, or irregularly shaped but accommodating the protruding parts of the thin strip or a combination thereof.

According to yet another embodiment of the present disclosure, at least one circumferential slot, is created on the inner shaft to increase flexibility of the catheter in area where the implant holder is located. The ends of the circumferential slots are not connected. The circumferential slots may partially overlap each other circumferentially or longitudinally.

According to yet another embodiment of the present disclosure, the thin strip covers the peripheral surface of the implant holder from the joined end till the pin or till the distal end of the implant holder with an eyelet on its peripheral surface to provide an access site that allows the thin strip to move without hinderance from the pin. In some embodiments, the size of the access site can extend along the length of the thin strip, and it can be of various shapes as well. Similarly, the thin strips can be of various sizes and shapes while conforming with the implant holder and the capsule in the compressed or normal state.

According to yet another embodiment of the present disclosure, the thin strip has at least one step change reduction in the thickness of the thin strip along the length of the cantilever or at least one step change curvature while maintaining the thickness of the thin strip along the length of the cantilever. The step change shape may be in shape of a stair or in curved shape or in a tapered shape. The step change shape allows to provide a push force to the engaging part of the implant frame, at the time of detachment.

According to yet another embodiment of the present disclosure, the implant may be, but not limited to, a stent, a valve, a mesh, a balloon, a patch, a drug-containing matrix, a shunt, or a combination thereof.

According to yet another embodiment of the present disclosure, the pin can be of various sizes and shapes, specifically selected from, but not limited to, the rectangular, circular, D-shaped, ovel, hexagonal, pentagonal, octagonal, and triangular configurations.

The materials used for fabricating such cantilever is selected from, but not limited to, stainless steel, nitinol, polyamide, polypropylene, Acrylonitrile butadiene styrene and a combination thereof.

According to yet another embodiment of the present disclosure, the implant holder comprises at least one radiopaque marker. The radiopaque marker is situated on the peripheral surface of the implant holder and its components including, but not limited to, the pin, the thin strip, the second thin strip, the third thin strip or a combination thereof.

According to yet another embodiment of the present disclosure, shape of the radiopaque marker present on the percutaneous catheter is selected from a circle, rectangular, square, oval, hexagonal, oblong, star-shaped, diamond-shaped, a circumferential ring, an irregular-shaped circumferential ring, an incomplete circumferential ring, an incomplete irregular circumferential ring or a combination thereof.

According to yet another embodiment of the present disclosure, the implant is used in treating any abnormality or in any medical procedure related to heart, kidney, lever, brain, pancreas, lungs, digestive system, endovascular system, any tract, duct or any conduit in animal or human body. More specifically, the implant can be deployed in an artery, vein, heart valves, esophageal duct, bile duct, urinary tract, alimentary tract, tracheobronchial tree, cerebral aqueduct or genitourinary system of an animal or human body.

In addition, the present subject matter also envisages a method for fabricating the implant holder as explained above. For the manufacturing of the implant holder, the method requires loading of a medically clean and approved workpiece in a designing instrument. According to one example of the present subject matter, the workpiece can be in shape of a hollow circular tube, or a solid cylinder, or a sheet. In some embodiments, the workpiece is prepared from a composition in powder form or prepared from a composition in liquid form. Then the required design of the implant holder is set-up or uploaded in the designing instrument, such as a computer-numerical controlled (CNC) machine for manufacturing. Subsequently, the required design is carved out of the workpiece to fabricate the implant holder. In one example, the fabrication technique used in the designing instrument is selected from laser fabrication, chemical-etching, mechanical machining, chemical machining, metal injection molding, vacuum casting, milling, photochemical-etching, electro-discharge machining, 3D-printing technique, additive manufacturing technique or a combination thereof. For instance, the implant holder is fabricated by slitting a metallic or polymeric hollow circular tube with a laser beam, the laser beam following a predefined cutting contour to produce the design of the implant holder. Alternatively, the implant holder is be manufactured using 3D printing technique or additive manufacturing. Once the implant holder has been manufactured, the undesired material is removed from the surface of the implant holder for finishing. The cleaned and finished implant holder can then be polished or coated with an appropriate coating. For example, it can be coated with an anti-reactive agent which prevents it from reacting with the atmosphere where either the implant is stored or deployed. Additionally, or alternatively, the implant holder can be covered with a medicinal substance or radiopaque substance, depending on the purpose, mode, and location of deployment of the implant holder.

3D printing technique can be selected from but not limited to Stereolithography (SLA), Digital light processing (DLP), Fused deposition modelling (FDM), Selective laser sintering (SLS), Selective laser melting (SLM), Electronic beam melting (EBM), Laminated object manufacturing (LOM), Polyjet technology or a combination of thereof.

By combining different materials and design variations explained above, a variety of configurations can be obtained with varying structure-property relationships.

Now, referring to the figures, wherein the elements are labelled with like numerals throughout the several Figures. Further, reference is made to the accompanying drawings which form a part hereof, and in which is shown by way of illustration a specific embodiment in which the invention may be practiced. It is to be understood that other embodiments may be utilized, and structural changes may be made without departing from the scope of the present invention.

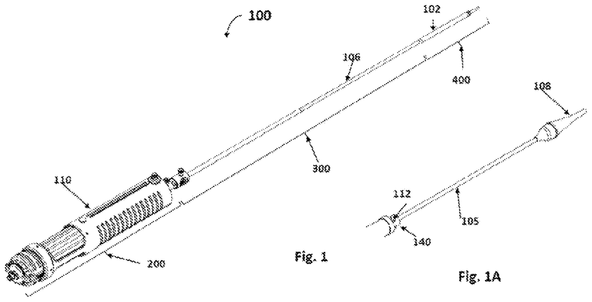

Fig. 1 and fig. 1A represent, according to an embodiment of the present disclosure, a typical catheter delivery system. The catheter delivery system (100) comprises a distal section (400), a middle section (300), and a proximal section (200). The proximal section (200) remains outside the human body and comprises a handle (110) to control the movements at the distal section (400) of the catheter. The distal section comprises a tip (108), a guidewire shaft (105), an implant holder (140) and a capsule (102). The middle section (300) is connected proximally with the handle housing and distally it connects to the distal section (400). The middle section (300) mainly comprises a catheter shaft (106) that connects to the capsule (102) in the distal section (400).

The capsule (102) provides an inside space where the implant is loaded in compressed form and the capsule (102) helps in retaining the implant in compressed form. The implant is situated over the guidewire shaft (105) and in between the tip (108) and the implant holder (140). The implant holder (140) is fixed on the inner shaft (104) and situated inside the capsule (102) at a proximal end of the distal section (400). The implant holder (140) has a plurality of pins ( 202, 302, 402, 502, 602, 702, 802) to get engaged with frame of the implant.

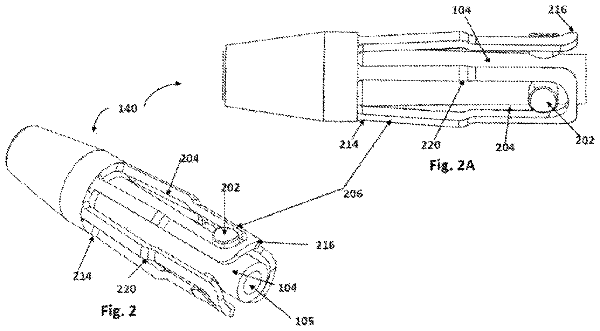

Referring to Fig. 2 and fig. 2A, according to an embodiment of the present disclosure, a thin strip (206), that is designed as a cantilever, has a one end (214) attached to a proximal end of the implant holder and the other end (216) is free. The thin strip (206) covers, partially, the peripheral surface of the implant holder from the one end till the pin (202) or till the distal end of the implant holder while having an elongated slot (204) on its peripheral surface to provide a space to the pin (202) while the thin strip (206) moves in radial direction. Further, optionally, the thin strip (206) has at least one step change (220) in the thin strip along its length.

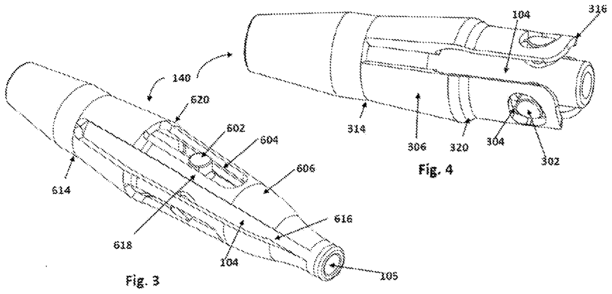

Referring to Fig. 3, according to another embodiment of the present disclosure, both the ends, the one end (614) and the other end (616) of the thin strip (606) are fixed to the proximal end and the distal end of the implant holder (140) respectively. However, the middle portion (618) of the thin strip (606) is not attached to any surface. This gives the thin strip (606) a bell-shaped configuration where a part of the middle portion (618) of the thin strip or the formed bell is near the pin (602). Due to the formed bell, fixed ends, and elastic property of the thin strip; the thin strip (606) behaves similar to a leaf spring. Further, optionally, the thin strip (606) has at least one step change (620) in the thin strip along its length.

Referring to Fig. 4, according to yet another embodiment of the present disclosure, a thin strip (306), that is designed as a cantilever, has a one end (314) attached to a proximal end of the implant holder and the other end (316) is free. The thin strip (306) covers, partially, the peripheral surface of the implant holder from the one end till the pin (302) or till the distal end of the implant holder while having an eyelet (304) on its peripheral surface to provide a space to the pin (302) while the thin strip (306) moves in radial direction. Further, optionally, the thin strip (306) has at least one step change (320) in the thin strip along its length.

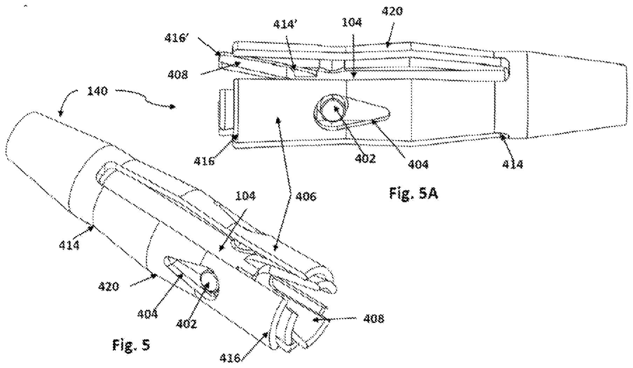

Referring to Fig. 5 and 5A, according to yet another embodiment of the present disclosure, the thin strip (406) superimposes a second thin strip (408) partially or completely. Similar to the thin strip (406), the one end (414') of the second thin strip (408) is connected to the peripheral surface of the implant holder and the other end (416') is free. The thin strip (406) covers, partially, the peripheral surface of the implant holder from the one end till the pin (402) or till the distal end of the implant holder while having an eyelet (404) on its peripheral surface to provide a space to the pin (402) while the thin strip (406) moves in radial direction. Further, optionally, the thin strip (406) has at least one step change (420) in the thin strip along its length.

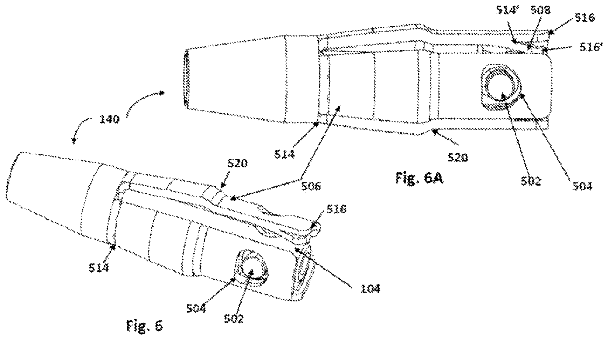

Referring to Fig. 6 and 6A, according to yet another embodiment of the present disclosure, a third thin strip (508) is attached to the thin strip (506) in a nested configuration. In nested configuration, the one end (514') of the third thin strip (508) is connected to the thin strip and the other end (516') extends in space between this strip and the peripheral surface of the implant holder (140). The thin strip (506) covers, partially, the peripheral surface of the implant holder from the one end till the pin (502) or till the distal end of the implant holder while having an eyelet (504) on its peripheral surface to provide a space to the pin (502) while the thin strip (506) moves in radial direction. Further, optionally, the thin strip (506) has at least one step change (520) in the thin strip along its length.

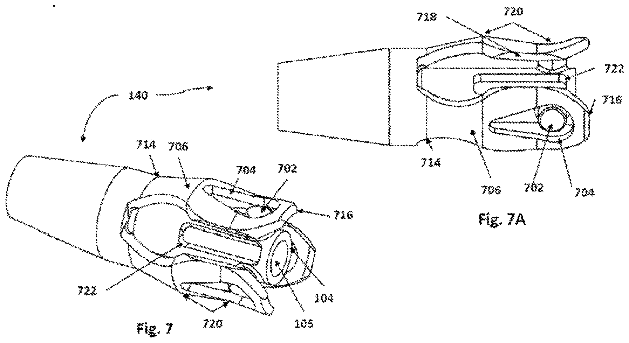

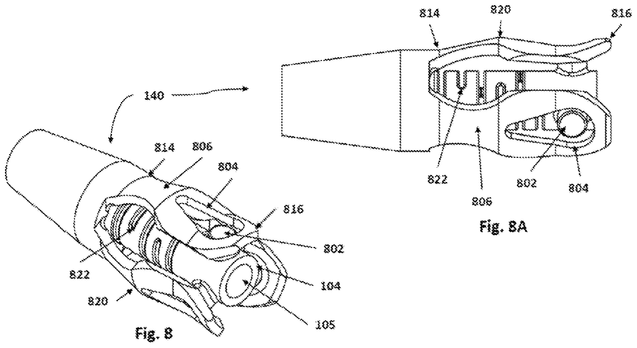

Referring to Fig. 7 and 7A, according to yet another embodiment of the present disclosure, the one end (714) of the thin strip (706) is attached to proximal side of the implant holder (140) and the other end (716) is free to move in radial direction based on compressive force applied or removed by the capsule. The thin strip (706) covers, partially, the peripheral surface of the implant holder from the one end till the pin (702) or till the distal end of the implant holder while having an eyelet (704) on its peripheral surface to provide a space to the pin (702) while the thin strip (706) comes down in radial direction. Further, the thin strip (706) has at least one step change (720) in the thin strip along its length. Due to the step-changes (720), the overall profile of the thin strip (706) increases in radial direction. Hence, a slot (722) is created in the inner shaft (104) to partially accommodate the middle portion (718) of the thin strip, while being compressed by the capsule, resulting in reduction of the overall profile of the thin strip (706) in radial direction.
Referring to Fig. 8 and 8A, according to yet another embodiment of the present disclosure, the one end (814) of the thin strip (806) is attached to proximal side of the implant holder (140) and the other end (816) is free to move in radial direction based on compressive force applied or removed by the capsule. The thin strip (806) covers, partially, the peripheral surface of the implant holder from the one end till the pin (802) or till the distal end of the implant holder while having an eyelet (804) on its peripheral surface to provide a space to the pin (802) while the thin strip (806) comes down in radial direction. The thin strip (806) has at least one step change (820) in the thin strip along its length. Further, at least one circumferential slot (822) is created on the inner shaft (104) to increase flexibility of the catheter in area where the implant holder (140) is located.

In the above description, for purpose of explanation, specific details are set forth in order to provide an understanding of the present disclosure. It will be apparent, however, to one skilled in the art that the present disclosure may be practiced without these details. One skilled in the art will recognize that embodiments of the present disclosure, one of which is described below, may be incorporated into a number of systems. Further, structures and devices shown in the figures are illustrative of exemplary embodiment of the present disclosure and are meant to avoid obscuring the present disclosure.

### List of reference Numerals

Capsule - 102
Inner shaft - 104
Guidewire shaft - 105
Catheter shaft - 106
Tip - 108
Handle - 110
Elongated slot - 204, 604
Implant holder - 140
Pin - 202, 302, 402, 502, 602, 702, 802
Thin strip - 206, 306, 406, 506, 606, 706, 806
Eyelet - 304, 404, 504, 704, 804
One end - 214, 414, 414', 514, 514', 714, 614, 814
Other end - 216, 416, 416', 516, 516', 716, 816, 616
Step-change - 220, 320, 420, 520, 620, 720, 820
Proximal section - 200
Middle section - 300
Distal section - 400
Second thin strip - 408
Third thin strip - 508
Middle portion - 618, 718
Slot - 722
Circumferential slot - 822

## Claims

1. An implant detaching mechanism to detach an implant from an implant holder, comprising:
a catheter having an inner shaft (104), a catheter shaft (106), a capsule (102), and a handle (110);
the implant holder (140) that is attached to the inner shaft (104), the implant holder having at least one pin (202; 302; 402; 502; 602; 702; 802) on its peripheral surface to engage an implant to the implant holder, the catheter shaft (106) being movable in the longitudinal direction, the capsule (102) being attached to the distal end of the catheter shaft, and the capsule covering and uncovering the implant holder and at least the engaged implant partially upon movement of the catheter shaft; and
a thin strip (206; 306; 406; 506; 606; 706; 806), elastic in nature, is attached to the implant holder (140) and placed between the peripheral surface of the implant holder and the engaged implant;
wherein, on moving the capsule (102) to cover the implant holder (140), a compressive force is applied on the thin strip (206; 306; 406; 506; 606; 706; 806) and the engaged implant;
wherein, on moving the capsule (102) to uncover the implant holder (140), release of potential energy stored in the thin strip (206; 306; 406; 506; 606; 706; 806) causes detachment of the engaged implant from the pin (202; 302; 402; 502; 602; 702; 802);
and wherein the thin strip (206; 306; 406; 506; 606; 706; 806) has an eyelet (304; 404; 504; 704; 804) or an elongated slot (204) on its peripheral surface to accommodate overlapping portion of the pin (202; 302; 402; 502; 602; 702; 802) on compression due to the movement of the capsule (102).

2. The implant detaching mechanism as claimed in claim 1, wherein the thin strip (206; 306; 406; 506; 606; 706; 806) in the implant holder (140) is attached towards proximal side of the implant holder at an one end and another end is free.

3. The implant detaching mechanism as claimed in claim 1, wherein the thin strip (206; 306; 406; 506; 606; 706; 806) in the implant holder (140) is attached towards proximal side of the implant holder at the one end and the other end is attached towards distal side of the implant holder and length of the thin strip being higher than the distance between the attached ends and causes the thin strip to take a curved configuration.

4. The implant detaching mechanism as claimed in claim 1, wherein the thin strip (206; 306; 406; 506; 606; 706; 806) in the implant holder (140) has at least a length sufficiently long to reach the closest peripheral surface of the pin ( 202; 302; 402; 502; 602; 702; 802), to reach the center point of the pin, to reach the farthest peripheral surface of the pin or to surpass the pin.

5. The implant detaching mechanism as claimed in claim 1, wherein the implant holder (140) has a plurality of pins (202; 302; 402; 502; 602; 702; 802) attached to the implant holder at equal or unequal distance in circumferential direction.

6. The implant detaching mechanism as claimed in claim 1, wherein the thin strip (206; 306; 406; 506; 606; 706; 806) in the implant holder (140) has at least a step-change reduction (220; 320; 420; 520; 620; 720; 820) in the thickness along its length or along its circumference.

7. The implant detaching mechanism as claimed in claim 1, wherein the thin strip (206; 306; 406; 506; 606; 706; 806) in the implant holder (140) has at least a step-change (220; 320; 420; 520; 620; 720; 820) in the curvature along its length or along its circumference.

8. The implant detaching mechanism as claimed in claim 1, wherein the thin strip (206; 306; 406; 506; 606; 706; 806) has at least one second thin strip (408) attached to the thin strip.

9. The implant detaching mechanism as claimed in claim 1, wherein the implant holder (140) has a plurality of thin strips (206; 306; 406; 506; 606; 706; 806) attached to the implant holder and disposed parallel to each other.

10. The implant detaching mechanism as claimed in claim 1, wherein the implant holder (140) is attached to the inner shaft (104), wherein the inner shaft (104) has at least one slot (722) extending longitudinally or circumferentially, and wherein the shape of the slot (722) is selected from rectangular, circular, helical, oval, or irregularly shaped configuration.

11. The implant detaching mechanism as claimed in claim 10, wherein at least one edge of the thin strip (206; 306; 406; 506; 606; 706; 806) is accommodated in the slot (722).

12. The implant detaching mechanism as claimed in any one of the preceding claims, wherein the implant is selected from a stent, a valve, a mesh, a balloon, a patch, a drug-containing matrix, a shunt, a vena cava filter, a vascular graft, a stent graft or a combination thereof.

13. The implant detaching mechanism as claimed in any one of the preceding claims, wherein the pin (202; 302; 402; 502; 602; 702; 802) on the implant holder (140) has a cross-sectional shape selected from rectangular, circular, D-shaped, oval, hexagonal, pentagonal, octagonal, triangular configuration and a combination thereof.

14. The implant detaching mechanism as claimed in any one of the preceding claims, wherein the implant holder (140) is made of a biocompatible material selected from a group of polymers, metals, alloys, non-metals, biodegradable materials, bioresorbable materials or a combination of thereof.

15. The implant detaching mechanism as claimed in any one of the preceding claims, wherein the implant holder (140) has at least one radiopaque marker on its peripheral surface.

## Patentansprüche

1. Implantatablösemechanismus zum Lösen eines Implantats von einem Implantathalter, umfassend:
einen Katheter, der einen inneren Schaft (104), einen Katheterschaft (106), eine Kapsel (102) und einen Griff (110) aufweist;
den Implantathalter (140), der an dem inneren Schaft (104) angebracht ist, wobei der Implantathalter mindestens einen Stift (202; 302; 402; 502; 602; 702; 802) an seiner Umfangsfläche aufweist, um ein Implantat mit dem Implantathalter einzugreifen, wobei der Katheterschaft (106) in Längsrichtung beweglich ist, die Kapsel (102) an dem distalen Ende des Katheterschafts angebracht ist und die Kapsel den Implantathalter und mindestens das eingegriffene Implantat bei Bewegung des Katheterschafts teilweise abdeckt und freigibt; und
ein dünner Streifen (206; 306; 406; 506; 606; 706; 806) elastischer Art wird an dem Implantathalter (140) angebracht und zwischen der Umfangsfläche des Implantathalters und dem eingesetzten Implantat platziert;
wobei bei Bewegen der Kapsel (102) zum Abdecken des Implantathalters (140) eine Druckkraft auf den dünnen Streifen (206; 306; 406; 506; 606; 706; 806) und das eingegriffene Implantat ausgeübt wird;
wobei bei Bewegen der Kapsel (102) zum Freilegen des Implantathalters (140) ein Freisetzen der in dem dünnen Streifen (206; 306; 406; 506; 606; 706; 806) gespeicherten potentiellen Energie eine Ablösung des eingegriffenen Implantats von dem Stift (202; 302; 402; 502; 602; 702; 802) bewirkt;
und wobei der dünne Streifen (206; 306; 406; 506; 606; 706; 806) eine Öse (304; 404; 504; 704; 804) oder einen länglichen Schlitz (204) an seiner Umfangsfläche aufweist, um einen überlappenden Abschnitt des Stifts (202; 302; 402; 502; 602; 702; 802) bei einer Kompression aufgrund der Bewegung der Kapsel (102) aufzunehmen.

2. Implantatablösemechanismus nach Anspruch 1, wobei der dünne Streifen (206; 306; 406; 506; 606; 706; 806) in dem Implantathalter (140) an einem Ende zu einer proximalen Seite des Implantathalters hin befestigt ist und das andere Ende frei ist.

3. Implantatablösemechanismus nach Anspruch 1, wobei der dünne Streifen (206; 306; 406; 506; 606; 706; 806) in dem Implantathalter (140) an einem Ende zu der proximalen Seite des Implantathalters hin befestigt ist und das andere Ende zu der distalen Seite des Implantathalters hin befestigt ist und die Länge des dünnen Streifens größer ist als der Abstand zwischen den befestigten Enden und bewirkt, dass der dünne Streifen eine gekrümmte Konfiguration annimmt.

4. Implantatablösemechanismus nach Anspruch 1, wobei der dünne Streifen (206; 306; 406; 506; 606; 706; 806) in dem Implantathalter (140) mindestens eine Länge aufweist, die ausreichend lang ist, um die nächstgelegene Umfangsfläche des Stifts (202; 302; 402; 502; 602; 702; 802) zu erreichen, um den Mittelpunkt des Stifts zu erreichen, um die am weitesten entfernte Umfangsfläche des Stifts zu erreichen oder um den Stift zu überragen.

5. Implantatablösemechanismus nach Anspruch 1, wobei der Implantathalter (140) eine Vielzahl von Stiften (202; 302; 402; 502; 602; 702; 802) aufweist, die in Umfangsrichtung in gleichem oder ungleichem Abstand an dem Implantathalter angebracht sind.

6. Implantatablösemechanismus nach Anspruch 1, wobei der dünne Streifen (206; 306; 406; 506; 606; 706; 806) in dem Implantathalter (140) mindestens eine stufenweise Verringerung (220; 320; 420; 520; 620; 720; 820) der Stärke entlang seiner Länge oder entlang seines Umfangs aufweist.

7. Implantatablösemechanismus nach Anspruch 1, wobei der dünne Streifen (206; 306; 406; 506; 606; 706; 806) in dem Implantathalter (140) mindestens eine stufenweise Änderung (220; 320; 420; 520; 620; 720; 820) der Krümmung entlang seiner Länge oder entlang seines Umfangs aufweist.

8. Implantatablösemechanismus nach Anspruch 1, wobei der dünne Streifen (206; 306; 406; 506; 606; 706; 806) mindestens einen zweiten dünnen Streifen (408) aufweist, der an dem dünnen Streifen angebracht ist.

9. Implantatablösemechanismus nach Anspruch 1, wobei der Implantathalter (140) eine Vielzahl von dünnen Streifen (206; 306; 406; 506; 606; 706; 806) aufweist, die an dem Implantathalter angebracht und parallel zueinander angeordnet sind.

10. Implantatablösemechanismus nach Anspruch 1, wobei der Implantathalter (140) an dem inneren Schaft (104) befestigt ist, wobei der innere Schaft (104) mindestens einen Schlitz (722) aufweist, der sich in Längsrichtung oder in Umfangsrichtung erstreckt, und wobei die Form des Schlitzes (722) ausgewählt ist aus einer rechteckigen, kreisförmigen, spiralförmigen, ovalen oder unregelmäßig geformten Konfiguration.

11. Implantatablösemechanismus nach Anspruch 10, wobei mindestens ein Rand des dünnen Streifens (206; 306; 406; 506; 606; 706; 806) in dem Schlitz (722) untergebracht ist.

12. Implantatablösemechanismus nach einem der vorherigen Ansprüche, wobei das Implantat ausgewählt ist aus einem Stent, einer Klappe, einem Netz, einem Ballon, einem Pflaster, einer arzneimittelhaltigen Matrix, einem Shunt, einem Vena-Cava-Filter, einem Gefäßgraft, einem Stentgraft oder einer Kombination davon.

13. Implantatablösemechanismus nach einem der vorherigen Ansprüche, wobei der Stift (202; 302; 402; 502; 602; 702; 802) an dem Implantathalter (140) eine Querschnittsform aufweist, die ausgewählt ist aus einer rechteckigen, kreisförmigen, D-förmigen, ovalen, hexagonalen, fünfeckigen, achteckigen, dreieckigen Konfiguration und einer Kombination davon.

14. Implantatablösemechanismus nach einem der vorherigen Ansprüche, wobei der Implantathalter (140) aus einem biokompatiblen Material gefertigt ist, das ausgewählt ist aus einer Gruppe von Polymeren, Metallen, Legierungen, Nichtmetallen, biologisch abbaubaren Materialien, bioresorbierbaren Materialien oder einer Kombination davon.

15. Implantatablösemechanismus nach einem der vorherigen Ansprüche, wobei der Implantathalter (140) mindestens eine röntgendichte Markierung an seiner Umfangsfläche aufweist.

## Revendications

1. Mécanisme de détachement d'implant pour détacher un implant d'un porte-implant, comprenant :
un cathéter ayant une tige interne (104), une tige de cathéter (106), une capsule (102) et une poignée (110) ;
le porte-implant (140) qui est fixé à la tige interne (104), le porte-implant ayant au moins un ergot (202 ; 302 ; 402 ; 502 ; 602 ; 702 ; 802) sur sa surface périphérique pour mettre un implant en prise avec le porte-implant, la tige de cathéter (106) étant mobile dans la direction longitudinale, la capsule (102) étant fixée à l'extrémité distale de la tige de cathéter, et la capsule recouvrant et découvrant le porte-implant et au moins partiellement l'implant en prise lors du mouvement de la tige de cathéter ; et
une bande mince (206 ; 306 ; 406 ; 506 ; 606 ; 706 ; 806), de nature élastique, est fixée au porte-implant (140) et placée entre la surface périphérique du porte-implant et l'implant en prise ;
dans lequel, en déplaçant la capsule (102) pour recouvrir le porte-implant (140), une force de compression est appliquée sur la bande mince (206 ; 306 ; 406 ; 506 ; 606 ; 706 ; 806) et sur l'implant en prise ;
dans lequel, en déplaçant la capsule (102) pour découvrir le porte-implant (140), la libération de l'énergie potentielle stockée dans la bande mince (206 ; 306 ; 406 ; 506 ; 606 ; 706 ; 806) entraîne le détachement de l'implant en prise de l'ergot (202 ; 302 ; 402 ; 502 ; 602 ; 702 ; 802) ;
et dans lequel la bande mince (206 ; 306 ; 406 ; 506 ; 606 ; 706 ; 806) comporte un oeillet (304 ; 404 ; 504 ; 704 ; 804) ou une fente allongée (204) sur sa surface périphérique pour accueillir la partie d'ergot (202 ; 302 ; 402 ; 502 ; 602 ; 702 ; 802) en chevauchement lors de la compression due au mouvement de la capsule (102).

2. Mécanisme de détachement d'implant selon la revendication 1, dans lequel la bande mince (206 ; 306 ; 406 ; 506 ; 606 ; 706 ; 806) dans le porte-implant (140) est fixée vers le côté proximal du porte-implant à une extrémité et une autre extrémité est libre.

3. Mécanisme de détachement d'implant selon la revendication 1, dans lequel la bande mince (206 ; 306 ; 406 ; 506 ; 606 ; 706 ; 806) dans le porte-implant (140) est fixée vers le côté proximal du porte-implant à une extrémité et l'autre extrémité est fixée vers le côté distal du porte-implant et la longueur de la bande mince étant supérieure à la distance entre les extrémités fixées et amène la bande mince à prendre une configuration incurvée.

4. Mécanisme de détachement d'implant selon la revendication 1, dans lequel la bande mince (206 ; 306 ; 406 ; 506 ; 606 ; 706 ; 806) dans le porte-implant (140) a au moins une longueur suffisante pour atteindre la surface périphérique la plus proche de l'ergot (202 ; 302 ; 402 ; 502 ; 602 ; 702 ; 802), pour atteindre le point central de l'ergot, pour atteindre la surface périphérique la plus éloignée de l'ergot ou pour dépasser l'ergot.

5. Mécanisme de détachement d'implant selon la revendication 1, dans lequel le porte-implant (140) a une pluralité d'ergots (202 ; 302 ; 402 ; 502 ; 602 ; 702 ; 802) fixés au porte-implant à une distance égale ou inégale dans la direction circonférentielle.

6. Mécanisme de détachement d'implant selon la revendication 1, dans lequel la bande mince (206 ; 306 ; 406 ; 506 ; 606 ; 706 ; 806) dans le porte-implant (140) présente au moins une réduction par paliers (220 ; 320 ; 420 ; 520 ; 620 ; 720 ; 820) de l'épaisseur sur sa longueur ou sur sa circonférence.

7. Mécanisme de détachement d'implant selon la revendication 1, dans lequel la bande mince (206 ; 306 ; 406 ; 506 ; 606 ; 706 ; 806) dans le porte-implant (140) présente au moins une variation par paliers (220 ; 320 ; 420 ; 520 ; 620 ; 720 ; 820) de la courbure sur sa longueur ou sur sa circonférence.

8. Mécanisme de détachement d'implant selon la revendication 1, dans lequel la bande mince (206 ; 306 ; 406 ; 506 ; 606 ; 706 ; 806) a au moins une seconde bande mince (408) fixée à la bande mince.

9. Mécanisme de détachement d'implant selon la revendication 1, dans lequel le porte-implant (140) a une pluralité de bandes minces (206 ; 306 ; 406 ; 506 ; 606 ; 706 ; 806) fixées au porte-implant et disposées parallèlement les unes aux autres.

10. Mécanisme de détachement d'implant selon la revendication 1, dans lequel le porte-implant (140) est fixé à la tige interne (104), dans lequel la tige interne (104) a au moins une fente (722) s'étendant longitudinalement ou circonférentiellement, et dans lequel la forme de la fente (722) est choisie parmi une configuration rectangulaire, circulaire, hélicoïdale, ovale ou de forme irrégulière.

11. Mécanisme de détachement d'implant selon la revendication 10, dans lequel au moins un bord de la bande mince (206 ; 306 ; 406 ; 506 ; 606 ; 706 ; 806) est logé dans la fente (722).

12. Mécanisme de détachement d'implant selon l'une quelconque des revendications précédentes, dans lequel l'implant est choisi parmi une endoprothèse, une valve, un filet, un ballon, un patch, une matrice contenant un médicament, un shunt, un filtre de veine cave, un greffon vasculaire, un greffon d'endoprothèse ou une combinaison de ceux-ci.

13. Mécanisme de détachement d'implant selon l'une quelconque des revendications précédentes, dans lequel l'ergot (202 ; 302 ; 402 ; 502 ; 602 ; 702 ; 802) du porte-implant (140) a une forme de section transversale choisie parmi une configuration rectangulaire, circulaire, en forme de D, ovale, hexagonale, pentagonale, octogonale, triangulaire, et une combinaison de celles-ci.

14. Mécanisme de détachement d'implant selon l'une quelconque des revendications précédentes, dans lequel le porte-implant (140) est constitué d'un matériau biocompatible choisi dans un groupe de polymères, de métaux, d'alliages, de non-métaux, de matériaux biodégradables, de matériaux biorésorbables ou d'une combinaison de ceux-ci.

15. Mécanisme de détachement d'implant selon l'une quelconque des revendications précédentes, dans lequel le porte-implant (140) comporte au moins un marqueur radio-opaque sur sa surface périphérique.
